# EUROPÄISCHE PATENTSCHRIFT

(11) **EP 2 964 163 B1**
(45) Veröffentlichungstag und Bekanntmachung des Hinweises auf die Patenterteilung: **08.05.2019**
(21) Anmeldenummer: 14709881.8
(22) Anmeldetag: 10.03.2014
(51) Int. Cl.: A61F 5/01

(54) **SCHIENENSYSTEM**
RAIL SYSTEM
SYSTÈME DE RAIL

(30) Priorität: 08.03.2013 DE 102013003936
(43) Veröffentlichungstag der Anmeldung: 13.01.2016
(73) Patentinhaber: Ottobock SE & Co. KGaA, 37115 Duderstadt (DE)
(72) Erfinder: SCHILLING, Matthias, 37345 Weißenborn-Lüderode (DE)
(74) Vertreter: Friedrich, Andreas
(86) Internationale Anmeldenummer: PCT/EP2014/000604
(87) Internationale Veröffentlichungsnummer: WO 2014/135281

(56) Entgegenhaltungen:
- BE-A- 469 500
- DE-A1- 1 541 298
- DE-A1-102007 013 438
- DE-U1- 20 204 917
- US-A- 1 265 635
- US-A- 2 463 124
- US-A- 2 567 577
- US-A- 3 913 570
- W. Beitz (hrsg.): "Dubbel, Taschenbuch für den Maschinenbau" In: "Dubbel, Taschenbuch für den Maschinenbau", 31. Dezember 2001 (2001-12-31), Springer, XP055133946, ISBN: 978-3-54-067777-2 Seiten G34-G37, Figur 46a-1

## Beschreibung

Die Erfindung betrifft ein orthopädietechnisches Schienensystem insbesondere für eine Orthese oder eine Prothese, das ein erstes Bauteil mit einem Aufnahmeelement und ein zweites Bauteil mit einem Verbindungsende zum Verbinden mit dem Aufnahmeelement aufweist, wobei das Aufnahmeelement zwei einander gegenüberliegende Anschlagswände aufweist, das Verbindungselement zwei einander gegenüberliegende Seitenwände aufweist und das Aufnahmeelement und das Verbindungsende durch wenigstens ein Verbindungselement miteinander verbindbar sind, sodass im verbundenen Zustand die Seitenwände an den Anschlagswänden anliegen, wobei Anschlagwände und die Seitenwände jeweils konisch aufeinander zulaufen und das Aufnahmeelement und das Verbindungsende stufenlos zueinander positionierbar sind. Beschrieben wird zudem ein Funktionsbauteil für ein orthopädietechnisches Schienensystem sowie ein Adapterelement zum Verbinden der beiden Bauteile des Schienensystems.

Viele Orthesen umfassen mehrere Bauelemente, die an unterschiedlichen Stellen am Körper des Trägers der Orthese angeordnet werden. So wird beispielsweise eine Knieorthese am Oberschenkel und am Unterschenkel des Trägers angeordnet. Diese einzelnen unterschiedlichen Bauteile werden durch ein gattungsgemäßes Schienensystem miteinander verbunden, wobei dieses Schienensystem beispielsweise frei schwenkbare oder einrastbare Gelenke, die mit entsprechenden Schienen verbunden werden, aber auch steife Verbindungen zwischen zwei Schienen umfassen kann. Um eine individuelle Anpassung der Orthese an den Körperbau des Trägers zu erreichen, müssen die einzelnen Schienen, die die verschiedenen Bauteile des Schienensystems und der Orthese miteinander verbinden, individuell für den Träger angefertigt oder abgelängt werden. Die Verbindung zweier Bauteile eines Schienensystems erfolgt herkömmlicherweise über eine Verbindung des Verbindungsendes eines Bauteiles, beispielsweise einer Schiene, mit einem Aufnahmeelement eines weiteren Bauteiles, das beispielsweise ebenfalls eine Schiene oder eines der bereits angesprochenen Gelenke sein kann.

Das Aufnahmeelement weist zwei einander gegenüberliegende parallele Seitenwände auf, zwischen die das Verbindungsende des jeweils anderen Bauteils positioniert wird. Um eine möglichst feste und sichere Verbindung sowie ein angenehmes und sicheres Tragegefühl für den Träger der Orthese zu erreichen, ist es notwendig, eine spielfreie Verbindung der einzelnen Bauteile miteinander zu erreichen. Dies ist nicht nur bei der endgültigen Orthese wichtig, sondern natürlich bereits im Zuge der Anprobearbeiten einer neuen Orthese zu gewährleisten. Herkömmlicherweise werden beispielsweise die Seitenwände und die Unterseite des Verbindungsendes des zweiten Bauteiles aufwändig nachgearbeitet, beispielsweise nachgefeilt oder nachgeschliffen, um eine möglichst gute Passgenauigkeit des Verbindungsendes mit dem Aufnahmeelement zu erreichen. Zusätzlich werden die beiden Bauteile über wenigstens zwei Schrauben miteinander verschraubt, wodurch eine besonders sichere Verbindung erreicht werden soll, da die Schrauben zumindest einen Teil der wirkenden Kräfte aufnehmen. Nachteilig ist, dass die Herstellung derart passgenauer Bauteile aufwändig, zeitintensiv und daher teuer ist und zudem bei der Anprobe der Orthese bei jeder Änderung der Abmessungen einzelner Bauteile die entsprechenden Verbindungsenden erneut nachgearbeitet werden müssen. Die Anprobe wird dabei ebenfalls langwierig und für den Träger der Orthese unangenehm, sofern er nicht bei der Anprobe auf eine spielfreie Verbindung verzichtet.

Alternativ oder zusätzlich dazu ist es bekannt, Ausgleichsplättchen oder Zwischenbauteile vorzusehen, die zwischen die beiden zu verbindenden Bauteile, beispielsweise in dem Aufnahmeelement des ersten Bauteils, eingelegt oder eingepasst werden, um Fertigungstoleranzen auszugleichen und für eine spielfreie Verbindung der beiden Bauteile zu sorgen. Dies hat den Nachteil, dass eine Vielzahl unterschiedlicher Ausgleichsplättchen vorzuhalten ist, um unterschiedliche Fertigungstoleranzen ausgleichen zu können. Diese Bauteile können verloren gehen, sodass eine dauerhafte spielfreie Verbindung insbesondere nachdem die einzelnen Bauteile gegebenenfalls häufiger voneinander gelöst wurden, nicht gewährleistet werden kann.

Für die endgültige Orthese ist es bekannt, zusätzlich oder alternativ zu einer besonderen Nacharbeitung des Verbindungsendes der verschiedenen Bauteile, die aneinander angepassten und gegebenenfalls verschraubten Bauteile zusätzlich zu verkleben. Dazu wird oftmals ein Spezialkleber verwendet, der in einem weiteren aufwändigen und daher zeit- und kostenintensiven Verfahrensschritt ausgehärtet werden muss. Nachteilig ist hierbei, dass die Verbindung nicht zerstörungsfrei wieder gelöst werden kann, sodass die einmal zusammengefügte Orthese nur sehr schwer geändert und an gegebenenfalls geänderte Voraussetzungen beim Patienten angepasst werden kann.

Um eine leichte Lösbarkeit der beiden Bauteile voneinander zu gewährleisten, ist aus dem Stand der Technik bekannt, beispielsweise eine Schiene mit einem Einsatzelement auszubilden, das in eine dafür vorgesehene Ausnehmung eingeführt wird. Dabei wird ein Verriegelungselement aus seiner Ruheposition gegen eine Federkraft eines Federelementes herausgedrückt und schnappt wieder in seine Ruheposition zurück, sobald das Einsatzelement seine endgültige Position erreicht hat. Durch das zurückgeschnappte Federelement wird die Verbindung verriegelt. Nachteilig ist jedoch auch hier, dass eine sehr exakte Passform der einzelnen Bauteile zu einander gewährleistet werden muss, damit die durch das zurückgeschnappte Federelement verriegelte Verbindung spielfrei ist.

Aus der US 3,913,570 ist ein Schienensystem bekannt, das bei Operationen für Arme und/oder Beine verwendet werden kann. Die einzelnen Elemente sind dabei über die gesamte Länge verjüngend ausgebildet, sodass zwei identische Schienen ineinander gesteckt werden können. Sie werden über Schrauben, die durch dafür vorgesehene Löcher in beiden zu verbindenden Schienen hindurchgeführt werden, miteinander verbunden. Eine spielfreie Verbindung wird dadurch nicht erreicht.

Die DE 202 04 917 U1 beschreibt eine einziehbare stangenartige Vorrichtung mit nicht kreisförmigem Querschnitt. Dazu werden Verschiebekeile verwendet, um eine feste Verbindung zweier miteinander zu verbindender Bauteile zu erreichen.

Aus der DE 15 41 298 A1 ist eine selbstzentrierende Verbindung zweier Bauteile bekannt.

Der Erfindung liegt folglich die Aufgabe zu Grunde, ein Schienensystem gemäß dem Oberbegriff des Anspruchs 1 so weiter zu entwickeln, dass eine spielfreie Verbindung einfach und schnell erreicht werden kann und dennoch eine einfache Lösbarkeit der Verbindung gewährleistet bleibt.

Die Erfindung löst die gestellte Aufgabe dadurch, dass das orthopädietechnische Schienensystem gemäß dem Oberbegriff des Anspruchs 1 sich dadurch auszeichnet, dass das Ausnahmeelement und das Verbindungsende stufenlos so miteinander verbindbar sind, dass sie auch in der Position miteinander verbindbar sind, in der sie spielfrei aneinander anliegen, wobei das Schienensystem zum Verbinden des Aufnahmeelementes mit dem Verbindungsende wenigstens ein Verbindungselement aufweist, das durch ein Langloch in dem ersten Bauteil oder dem zweiten Bauteil geführt ist. Dass jeweils zwei Wände konisch aufeinander zulaufen bedeutet, dass sich der Abstand der beiden Seitenwände und der beiden Anschlagswände jeweils zu einander in eine Richtung stetig verringert oder abschnittsweise konstant bleibt. Dies kann beispielsweise in einer Richtung parallel zu einer Längsrichtung des Bauteiles erfolgen oder beispielsweise in einer Richtung senkrecht dazu.

Dadurch wird erreicht, dass unabhängig von eventuell vorhandenen Ungenauigkeiten und Fertigungstoleranzen eine spielfreie Verbindung auch ohne ein zusätzliches Verkleben auch mit beispielsweise nur einem einzigen Verbindungselement erreicht werden kann. Dadurch, dass sowohl die Anschlagswände als auch die Seitenwände jeweils konisch aufeinander zulaufen und sich der Abstand zwischen beiden Anschlagswänden und der Abstand zwischen den beiden Seitenwänden folglich verringert, können die beiden miteinander zu verbindenden Bauteile so zu einander angeordnet werden, dass eine Anschlagswand und eine Seitenwand vollflächig aneinander anliegen. In dem Moment, wo die jeweils andere Anschlagswand mit der jeweils anderen Seitenwand in Berührung kommt, ist eine Rotation um eine Achse senkrecht zum Boden bzw. der Unterseite der beiden miteinander zu verbindenden Bauteile nicht mehr möglich. In diesem Fall ist ein einziges Verbindungselement ausreichend, um eine spielfreie Verbindung zu gewährleisten.

Die vollflächige Anlage kann insbesondere dann erreicht werden, wenn die eine Anschlagswand und die eine Seitenwand, die vollflächig aneinander anliegen sollen, grade verlaufen oder den gleichen Krümmungsradius aufweisen. Doch selbst bei unterschiedlichen Krümmungsradien der jeweiligen Wände lässt sich eine spielfreie Verbindung erreichen.

Erfindungsgemäß sind das Aufnahmeelement und das Verbindungsende stufenlos zueinander positionierbar. Durch die Kombination der konisch aufeinander zu laufenden Seiten- bzw. Anschlagswände und der Möglichkeit, das Aufnahmeelement und das Verbindungsende stufenlos zu einander zu positionieren und in jeder so erreichten Position auch miteinander zu verbinden, wird die der Erfindung zugrunde liegende Aufgabe besonders einfach gelöst. Dadurch, dass das Aufnahmeelement und das Verbindungsende stufenlos zu einander positionierbar und miteinander verbindbar sind, wird erreicht, dass beide Bauteile insbesondere auch in der Position miteinander zu verbinden sind, in der sie spielfrei aneinander anliegen. Dadurch wird auf konstruktiv besonders einfache Weise erreicht, dass aufgrund von Fertigungstoleranzen auftretende Ungenauigkeiten ausgeglichen werden können und es trotz dieser Ungenauigkeiten zu einer spielfreien Verbindung der beiden Bauteile kommen kann.

Bevorzugt weist das Aufnahmeelement einen Boden und das Verbindungsende eine Unterseite auf, wobei die Unterseite im verbundenen Zustand der beiden Bauteile an dem Boden anliegt. Dadurch, dass Boden und Unterseite im verbundenen Zustand ebenfalls aneinander anliegen, ist ein Einlegen von Austauschplättchen oder ähnlichen Austauschteilen nicht nötig. Durch das Lösen des Verbindungselementes, das beispielsweise eine Schraube sein kann, lässt sich das Aufnahmeelement von dem Verbindungsende und damit das erste Bauteil von dem zweiten Bauteil einfach und schnell lösen. Dies erlaubt eine sichere und spielfreie Verankerung der beiden Bauteile aneinander bereits bei der Anprobe und Anpassung der Orthese an den jeweiligen Körper des Patienten.

In einer besonders vorteilhaften Ausgestaltung der vorliegenden Erfindung handelt es sich bei dem Aufnahmeelement um einen Schienenkasten.

Erfindungsgemäß verfügt das Schienensystem zum Verbinden des Aufnahmeelementes mit dem Verbindungsende über wenigstens ein Verbindungselement. Es ist auch möglich, genau ein Verbindungselement zu verwenden. Dadurch wird eine besonders einfache Ausgestaltung erreicht.

Vorteilhafterweise weist das erste Bauteil eine erste Längsrichtung auf, wobei wenigstens eine Anschlagswand mit der ersten Längsrichtung einen von 0° verschiedenen Winkel einschließt. Die erste Längsrichtung ist dabei insbesondere die Richtung, in der das Verbindungsende des zweiten Bauteils in das Aufnahmeelement des ersten Bauteils eingeführt wird. Handelt es sich bei dem ersten Bauteil beispielsweise um eine Schiene, entspricht diese Längsrichtung vorteilhafterweise der Richtung, in der die Schiene ihre längste Ausdehnung hat. Der Vorteil der vorliegenden Erfindung tritt dabei bereits dann ein, wenn nur eine der Anschlagswände mit der ersten Längsrichtung einen von 0° verschiedenen Winkel einschließt. Die verbleibende zweite Anschlagswand kann dann in herkömmlicher Weise beispielsweise parallel zur Längsrichtung ausgeführt werden.

In einer vorteilhaften Ausgestaltung des Schienensystems weist das zweite Bauteil eine zweite Längsrichtung auf, wobei wenigstens eine Seitenwand mit der zweiten Längsrichtung einen von 0° verschiedenen Winkel einschließt. Die zweite Längsrichtung entspricht dabei oftmals der Richtung, in die das Verbindungsende des zweiten Bauteils in das Aufnahmeelement des ersten Bauteils eingeschoben wird. Auch in diesem Fall ist es ausreichend, wenn nur eine Seitenwand mit der zweiten Längsrichtung einen von 0° verschiedenen Winkel einschließt.

Als vorteilhaft hat sich jedoch herausgestellt, wenn beide Anschlagswände und/oder beide Seitenwände mit der jeweiligen ersten Längsrichtung bzw. zweiten Längsrichtung einen von 0° verschiedenen Winkel einschließen. Als besonders vorteilhaft hat sich dabei herausgestellt, wenn die Winkel zwischen den Anschlagswänden und der ersten Längsrichtung sowie zwischen den Seitenwänden und der zweiten Längsrichtung identisch ausgebildet sind.

Die identische Ausbildung der Winkel für die beiden Anschlagswände und für die beiden Seitenwände hat zur Folge, dass sowohl das erste als auch das zweite Bauteil in zwei unterschiedlichen Orientierungen verwendet werden kann. So ist beispielsweise das Verbindungsende in zwei um 180° zu einander verschwenkten Orientierungen in das Aufnahmeelement einführbar. Dadurch wird die Montage erleichtert, da beiden Bauteile nicht mehr "falsch" zusammengesetzt werden können. Zudem kann für die Herstellung des jeweiligen Bauteils für beide Wände das gleiche Werkzeug verwendet werden. Auf diese Weise ist es möglich, das Aufnahmeelement in einer Aufspannung zu fertigen, wodurch einerseits der Herstellungs- und Fertigungsaufwand reduziert wird und andererseits die Präzision erhöht wird, mit der die beiden Seitenwände relativ zu einander gefertigt werden können.

Sind auch die Winkel zwischen den beiden Anschlagswänden einerseits und den beiden Seitenwänden andererseits identisch, führt dies zu einer besonders guten Passgenauigkeit und einer insbesondere guten Reproduzierbarkeit der Verbindung. Damit ist sichergestellt, dass die beiden Längsrichtungen des ersten und des zweiten Bauteiles beispielsweise nach dem Verbinden der beiden Bauteile durch das Einführen des Verbindungsendes in das Aufnahmeelement parallel oder in einem vorbestimmten gewünschten Winkel zueinander verlaufen.

In einer bevorzugten Ausgestaltung des Schienensystems sind das Aufnahmeelement und das Verbindungsende in zwei senkrecht aufeinander stehenden Richtungen stufenlos zueinander positionierbar und miteinander verbindbar. Dadurch wird erreicht, dass unterschiedlichste Abweichungen und Fehlertoleranzen ausgeglichen werden können. Vorzugsweise erstrecken sich diese beiden Richtungen parallel zu der Unterseite des Verbindungsendes und zu dem Boden des Aufnahmeelementes.

Bei der Herstellung des Verbindungsendes des zweiten Bauteiles müssen die Seitenwände des Verbindungselementes beispielsweise mittels einer Fräse hergestellt werden. Eine mögliche Abweichung aufgrund von Ungenauigkeiten bei der Fertigung besteht in der sogenannten Winkelabweichung. Dies bedeutet, dass die gefertigte Seitenwand einen anderen als den gewünschten Sollwinkel beispielsweise mit der Längsrichtung des Bauteiles einschließt. Eine andere mögliche Abweichung besteht in der Position der Seitenwand, die beispielsweise durch zu viel oder zu wenig Abtrag durch die Fräse verschoben werden kann. In diesem Fall weist die Seitenwand beispielsweise den gewünschten Sollwinkel zur Längsrichtung des jeweiligen Bauteils auf, befindet sich jedoch an einer relativ zur Mittelachsen des Bauteils verschobenen Position. Eine tatsächliche Abweichung von der Sollform des Verbindungsendes wird in der Regel aus einer Kombination beider Abweichungen bestehen.

Dabei gilt es jedoch zu beachten, dass Positionsabweichungen durch zu viel oder zu wenig Abtrag von Material herkömmlicherweise deutlich häufiger und mit größerer Abweichung auftreten als dies bei Winkelabweichungen der Fall ist. Unabhängig von der auftretenden Abweichung kann insbesondere mit der in zwei unterschiedlichen Richtungen gewährleisteten Verschiebbarkeit immer eine spielfreie Verbindung erreicht werden, sofern die Abweichungen nicht über einen vorgegebenen Toleranzbereich hinausgehen.

Vorteilhafterweise ist das Aufnahmeelement lösbar mit dem ersten Bauteil verbunden. In diesem Fall können beispielsweise unterschiedliche Aufnahmeelemente vorgehalten werden, die für unterschiedliche Zwecke mit dem ersten Bauteil verbunden werden können. So ist es beispielsweise denkbar, unterschiedliche Aufnahmeelemente mit dem ersten Bauteil zu verbinden, die dafür sorgen, dass das mit dem ersten Bauteil zu verbindende zweite Bauteil in einem bestimmten Winkel zu dem ersten Bauteil verläuft. So kann beispielsweise der Winkel zwischen der ersten und der zweiten Längsrichtung der jeweiligen Bauteile zueinander eingestellt und auf die Bedürfnisse des jeweiligen Patienten angepasst werden.

Natürlich ist es auch von Vorteil, ein lösbares Verbindungsendebauteil vorzusehen, das mit einem Ende eines herkömmlichen Bauteiles, das nicht über ein Verbindungsende verfügt, verbindbar ist. Sowohl das Verbindungsendebauteil als auch ein separates Aufnahmeelement kann dann beispielsweise vor der Anprobe und Anpassung der Orthese mit einem Bauteil beispielsweise gemäß dem Stand der Technik verbunden werden. Das Verbindungsendebauteil verfügt über ein Verbindungsende in der bereits beschriebenen Form und kann mit diesem Verbindungsende folglich in ein Aufnahmeelement der bereits beschriebenen Form eingeführt und dort arretiert werden. So können auch Schienensystems nach dem Stand der Technik nachgerüstet werden, so dass auch für diese Systeme der erfindungsgemäße Vorteil nutzbar ist.

Ein vorteilhaftes Funktionsbauteil für ein orthopädietechnisches Schienensystem zeichnet sich dadurch aus, dass das Funktionsbauteil einen Aufnahmeraum zum Aufnehmen eines Endes des Bauteiles aufweist, der wenigstens eine Verschiebefläche und einen daran verschiebbar gelagerten Verschiebekeil aufweist.

Als besonders vorteilhaft hat sich dabei herausgestellt, dass das Ende des Bauteiles durch Verschieben des wenigstens einen Verschiebekeiles entlang der wenigstens einen Verschiebefläche festklemmbar ist, wenn es in dem Aufnahmeraum aufgenommen ist. Dazu ist es sinnvoll, den wenigstens einen Verschiebekeil in unterschiedlichen Positionen arretierbar auszugestalten. Diese unterschiedlichen Positionen sind vorteilhafterweise stufenlos einstellbar. Wird nun ein Ende eines Bauteils in dem Aufnahmeraum aufgenommen, kann der wenigstens eine Verschiebekeil entlang der wenigstens einen Verschiebefläche verschoben werden und sorgt so dafür, dass das Ende des Bauteils in dem Aufnahmeraum festgeklemmt wird. Dabei ist der wenigstens eine Verschiebekeil so weit verschoben worden, dass es zu einer spielfreien Verbindung zwischen den beiden Bauteilen kommt. In diesem Zustand ist der wenigstens eine Verschiebekeil arretierbar, sodass sowohl der Verschiebekeil als auch das Ende des Bauteils in dieser Position festgehalten werden. Natürlich können weitere Befestigungsmittel, beispielsweise Schrauben o.ä. Vorrichtungen vorhanden sein, um die so gefundene Position des Endes des Bauteils in dem Aufnahmeraum des Funktionsbauteiles zu stabilisieren.

Das Funktionsbauteil kann dabei beispielsweise ein Teil eines Gelenkes, eine Schiene eines orthopädietechnischen Schienensystems oder ein sonstiges anderes Bauteil sein.

In einer bevorzugten Ausgestaltung ist der wenigstens eine Verschiebekeil zum Festklemmen des Endes des Bauteiles in einer Richtung senkrecht zu einer Längsrichtung des Bauteiles verschiebbar. Auf diese Weise kann der Aufnahmeraum beispielsweise zu einer Richtung, beispielsweise oben, geöffnet ausgebildet sein, während der wenigstens eine Verschiebekeil an einem Deckelelement angeordnet ist, durch das diese Öffnung des Aufnahmeraums verschlossen werden kann. Dabei wird beispielsweise der wenigstens eine Verschiebekeil in einen Zwischenraum zwischen einer Seitenwand des Aufnahmeraumes und dem in dem Aufnahmeraum befindlichen Ende des Bauteils eingeführt, sodass mit tieferen Einführen des Keiles in diesen Zwischenraum das Ende des Bauteils so weit verschoben wird, bis es zwischen dem wenigstens einem Verschiebekeil und einem weiteren Bauteil eingeklemmt ist. Auch dieses weitere Bauteil kann ein weiterer Verschiebekeil sein, sodass das Ende des Bauteils zwischen zwei Verschiebekeilen einklemmbar ist. Alternativ dazu kann der Verschiebekeil auch als separates Bauteil vorliegen, das durch das Deckelelement in die gewünschte Position gedrückt wird.

Vorzugsweise schließt die Verschiebefläche mit einer Längsrichtung des Bauteils einen von 0° verschiedenen Winkel ein. Soll nun ein derartiges Aufnahmeelement oder ein Verbindungsendebauteil an einem der Bauteile befestigt werden, wird ein Ende des Bauteils in den Aufnahmeraum eingesetzt. Dabei sind das Ende des Bauteiles und der Verschiebekeil vorteilhafterweise derart aufeinander abgestimmt, dass sie über Kontaktflächen verfügen, mit denen sie aneinander anliegen, wobei diese Kontaktflächen parallel zueinander ausgebildet sind.

Durch eine Verschiebung des Verschiebekeils entlang der Verschiebefläche kann auch bei dieser Ausgestaltung das Bauteil in dem Aufnahmeraum eingeklemmt und angepasst werden und anschließend beispielsweise zusätzlich durch eine Verschraubung fixiert werden. Das Aufnahmeelement oder das Verbindungsendebauteil ist dann mit dem ersten Bauteil fest verbunden. Auch diese Verbindung kann vorteilhafterweise leicht lösbar ausgebildet werden. Die Verbindung zwischen dem jeweiligen Bauteil und einem Aufnahmeelement oder dem Verbindungsendebauteil kann jedoch bereits vor der Anprobe oder Anpassung der Orthese an den Patient erfolgen, sodass eine leichte Lösbarkeit in diesem Fall nicht von übergeordnetem Interesse ist. Als vorteilhaft hat sich herausgestellt, wenn es sich bei dem Funktionsbauteil um ein Aufnahmeelement oder ein Verbindungsendebauteil für ein hier beschriebenes orthopädietechnisches Schienensystem handelt.

Ein vorteilhaftes Adapterelement zum Verbinden des ersten Bauteils mit dem zweiten Bauteil eines Schienensystems, mit dem das erste Bauteil mit einem dem Aufnahmeelement gegenüberliegenden Ende an einem dem Verbindungsende gegenüberliegenden Ende des zweiten Bauteils verbunden werden kann, zeichnet sich dadurch aus, dass das Adapterelement zwei Aufnahmeräume zum Aufnehmen des Endes des ersten Bauteils und des Endes des zweiten Bauteils aufweist, die jeweils wenigstens eine Verschiebefläche und einen daran verschiebbar gelagerten Verschiebekeil aufweisen. Vorzugsweise schließen die Verschiebeflächen mit der jeweiligen Längsrichtung des ersten und des zweiten Bauteils einen von 0° verschiedenen Winkel ein. Wie bei dem bereits beschriebenen Aufnahmeelement und dem Verbindungsendebauteil können hier beide Bauteile mit dem Adapterelement verbunden werden. Dies geschieht über eine Verschiebung des jeweiligen Verschiebekeils, sodass das jeweilige Bauteil in dem Adapterelement eingeklemmt und anschließend beispielsweise durch vorhandene Verbindungselemente, wie Schrauben, befestigt wird. Ein derartiges Adapterelement ist insbesondere für die Fälle von Vorteil, in denen Schienen oder Bauteile miteinander verbunden werden sollen, die nicht über ein Verbindungsende mit konisch aufeinander zulaufenden Seitenwänden verfügen. Damit können auch Schienensysteme nach dem Stand der Technik mit dem Adapterelement ausgerüstet und so miteinander verbunden werden. Natürlich kann ein derartiges Adapterelement nicht nur für eine starre Verbindung zwischen zwei Bauteilen sorgen sondern auch unterschiedlichste Arten und Formen von Gelenken, beispielsweise Rastgelenke oder Gelenke mit Winkelbegrenzungen in unterschiedliche Richtungen ausgestattet werden.

Durch das separate Adapterelement ist es zudem möglich, insbesondere Längenanpassung besonders einfach durchführen zu können. Dabei kann von dem jeweiligen Bauteil insbesondere das Ende gekürzt werden, an dem sich keine konisch zulaufenden Seitenwände befinden. Dieses Ende wird in aus dem Stand der Technik bekannterweise gekürzt, sodass die Bauteile auf die gewünschte Länge angepasst werden können. Dabei muss das Ende insbesondere nach dem Kürzen nicht mit konisch zulaufenden Seitenwänden versehen werden, sodass die gewünschte Kürzung besonders einfach und schnell durch den Orthopädietechniker vor Ort vorgenommen werden kann. Dabei wird die Qualität der spielfreien Verbindung zwischen den einzelnen Bauteilen nicht beeinträchtigt.

In allen beschriebenen Ausführungsformen ist es vorteilhaft, wenn die Seitenwände und die Unterseite einen rechten Winkel einschließen. Vorteilhaft ist zudem, wenn auch die Anschlagswände und der Boden einen rechten Winkel einschließen. Natürlich sind jedoch auch andere Ausführungsformen denkbar.

Mithilfe einer Zeichnunq werden nachfolgend Ausführungsbeispiele der vorliegenden Erfindung näher erläutert.

Zeichnungen 1-10 zeigen Ausführungsbeispiele der vorliegenden Erfindung. Zeichnungen 11-21 zeigen weitere Verbindungen zweier Bauteile eines Schienensystems.

Es zeigt
- Figur 1: - ein Bauteil mit einem Verbindungsende,
- Figur 2: - ein Bauteil mit einem Schienenkasten,
- Figur 3: - die beiden Bauteile aus Figur 1 und 2 im verbundenen Zustand,
- Figur 4: - das Bauteil aus Figur 1 in unterschiedlichen Ansichten,
- Figur 5: - das Bauteil aus Figur 2 in unterschiedlichen Ansichten,
- Figur 6: - die verbundenen Bauteile aus Figur 3 in unterschiedlichen Ansichten,
- Figur 7: - ein Bauteil mit einem Verbindungsende und einen Schienenkasten,
- Figur 8: - die beiden Bauteile aus Figur 7 im verbundenen Zustand in unterschiedlichen Ansichten,
- Figur 9: - verbundene Bauteile eines Schienensystems in unterschiedlichen Ansichten,
- Figur 10: - zwei über ein Adapterelement verbundene Bauteile in unterschiedlichen Ansichten,
- Figuren 11 bis 14: - Verbindungen zweier Bauteile eines Schienensystems in einer schematischen 3D- und einer Schnittdarstellung,
- Figur 15: - die schematische Schnittdarstellung durch ein Bauteil mit einem Funktionsbauteil,
- Figuren 16 bis 19: - weitere Darstellungen miteinander verbundener Bauteile in einer schematischen 3D- und einer Schnittdarstellung und
- Figuren 20 und 21: - weitere Darstellungen einer Verbindungsmöglichkeit

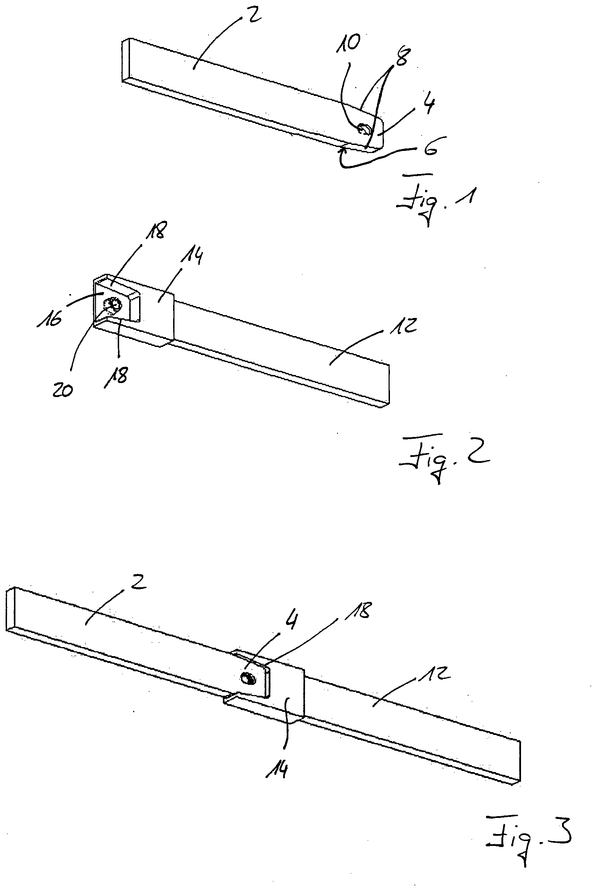

Figur 1 zeigt ein zweites Bauteil 2, an dessen einem Ende sich ein Verbindungsende 4 befindet. Das Verbindungsende 4 weist eine Unterseite 6 sowie zwei Seitenwände 8 auf. Man erkennt, dass die beiden Seitenwände 8 konisch aufeinander zulaufen, so dass in dem gezeigten Ausführungsbeispiel der Abstand der beiden Seitenwände 8 abnimmt. In dem Verbindungsende 4 befindet sich eine Bohrung 10, die als Langloch ausgebildet ist. Durch diese kann ein Verbindungselement geführt werden, um zwei Bauteile miteinander zu verbinden.

Figur 2 zeigt ein erstes Bauteil 12, an dessen einem Ende sich ein Aufnahmeelement 14 in Form eines Schienenkastens befindet. Dieser verfügt über einen Boden 16 sowie zwei Anschlagswände 18, die einander gegenüberliegen und konisch aufeinander zulaufen. Auch der Abstand der beiden Anschlagswände 18 zueinander verringert sich folglich. Zentral befindet sich eine Aufnahmeeinrichtung 20, die beispielsweise als Bohrung mit in dem Gewinde ausgebildet sein kann.

Figur 3 zeigt das erste Bauteil 12 und das zweite Bauteil 2 im ineinander gefügten Zustand. Man erkennt, dass das Verbindungsende 4 in den Schienenkasten aufgenommen ist. Dabei liegt die Unterseite 6 am Boden 16 an. Zudem sind die Seitenwände 8 des Verbindungsendes 4 mit den Anschlagswänden 18 des Schienenkastens in Kontakt. Die Bohrung 10 liegt fluchtend über der Aufnahmeeinrichtung 20, sodass ein hier eingefügtes Verbindungselement, das in Figur 3 nicht dargestellt ist, das erste Bauteil 12 und das zweite Bauteil 2 aneinander festlegt. Man erkennt, dass die Bohrung 10 als Langloch ausgebildet ist, sodass eine Verschiebbarkeit der beiden Bauteile 2, 12 relativ zueinander gegeben ist.

Dadurch, dass sowohl die Seitenwände 8 als auch die Anschlagswände 18 konisch aufeinander zulaufend ausgebildet sind, wird unabhängig von gegebenenfalls vorhandenen Ungenauigkeiten im Rahmen von Fertigungstoleranzen immer eine spielfreie Verbindung der beiden Bauteile 2, 12 miteinander gewährleistet.

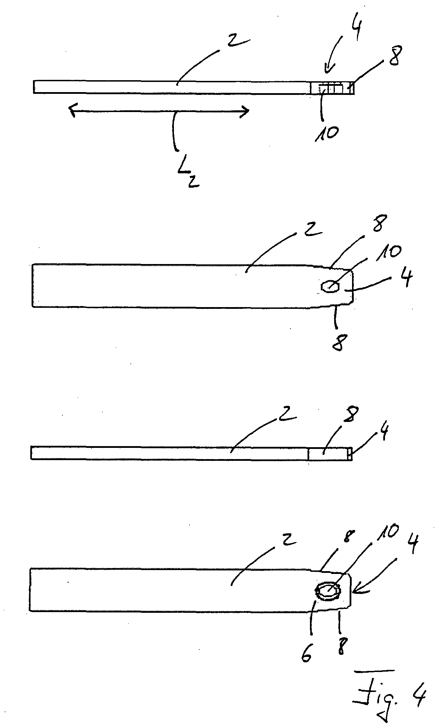

Figur 4 zeigt das zweite Bauteil 2 aus Figur 1 in unterschiedlichen Ansichten. Die oberste Darstellung zeigt eine Schnittdarstellung entlang einer zweiten Längsrichtung L₂. Im rechten Teil dieser Darstellung ist das Verbindungsende 4 mit einer abgewinkelten Seitenwand 8 zu erkennen. Hindurch verläuft die Bohrung 10.

Darunter befindet sich eine Draufsicht auf das zweite Bauteil 2 mit dem Verbindungsende 4 und der sich darin befindenden Bohrung 10. Auch hier sind die konisch aufeinander zulaufenden Seitenwände 8 dargestellt. Darunter befindet sich eine Seitenansicht des zweiten Bauteiles 2.

Die unterste Darstellung der Figur 4 zeigt das zweite Bauteil 2 in einer Ansicht von unten. Man erkennt hier folglich die Unterseite 6 des Verbindungsendes 4 sowie die beiden Seitenwände 8. Die Bohrung 10 ist in zwei unterschiedlichen Tiefen ausgeführt, wie dies bereits im oberen Bereich der Figur 4 gezeigten Schnittdarstellung zu erkennen ist. Ein hierin anzuordnendes Verbindungselement kann auf diese Weise beispielsweise mit einem Schraubkopf in dem zweiten Bauteil 2 versenkt werden.

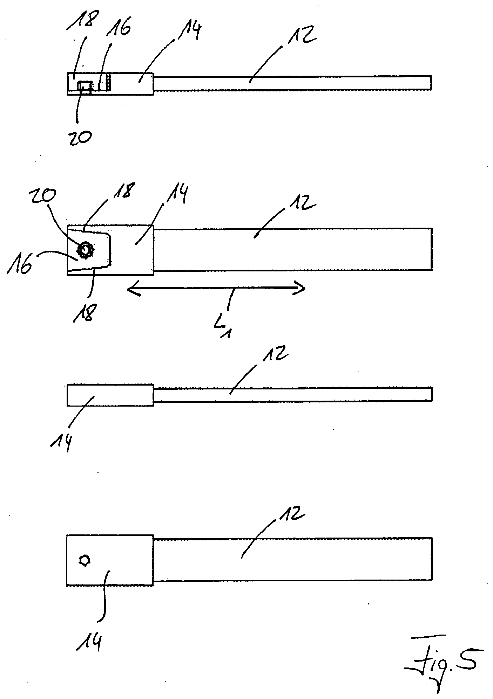

Figur 5 zeigt das erste Bauteil 12 in den gleichen Ansichten wie das erste Bauteil 2 in Figur 4 dargestellt ist. Ganz oben befindet sich folglich die Schnittdarstellung durch das erste Bauteil 12 und den Schienenkasten 14. Darunter ist eine Draufsicht dargestellt. In dieser ist besonders gut zu erkennen, dass die beiden Anschlagswände 18 konisch aufeinander zulaufend ausgebildet sind und in diesem Fall beide einen von 0° verschiedenen Winkel zu einer ersten Längsrichtung L₁ einschließen. In der darunter dargestellten Seitenansicht des ersten Bauteils 12 mit dem Schienenkasten 14 ist besonders gut zu erkennen, dass der für den Schienenkasten 14 benötigte Bauraum äußerst klein ausfallen kann. Da im gezeigten Ausführungsbeispiel nur eine Aufnahmeeinrichtung 20 für genau ein Verbindungselement vorgesehen ist, kann der Schienenkasten 14 gegenüber aus dem Stand der Technik bekannten Schienenkästen deutlich kleiner ausgebildet werden.

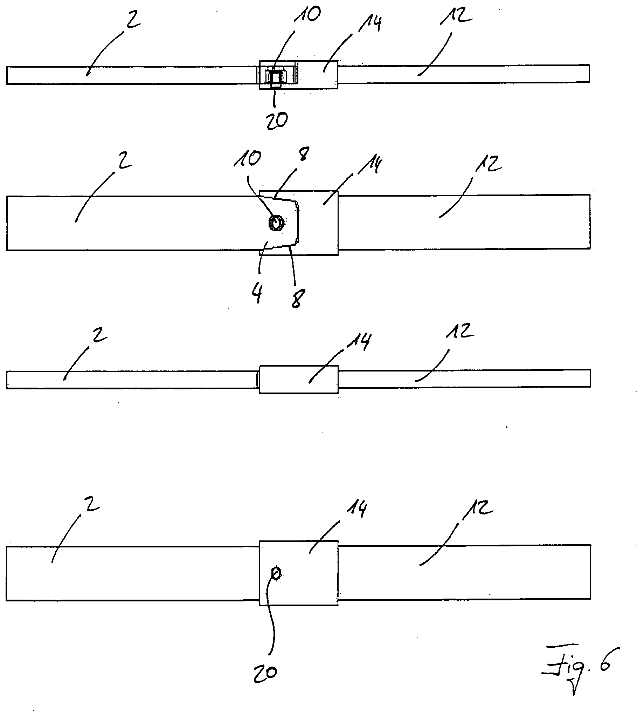

Figur 6 zeigt die beiden Bauteile 2, 12 im verbundenen Zustand in den bereits aus den Figuren 4 und 5 bekannten Darstellungen und Ansichten. Das Verbindungsende 4 des zweiten Bauteils 2 ist in den Schienenkasten 14 des ersten Bauteils 12 eingeführt. Die Seitenwände 8 liegen an den Anschlagswänden 18 und die Unterseite 6 am Boden 16 an. Durch diese Ausgestaltung wird eine baulich kleine und dennoch spielfreie und sichere Verbindung der beiden Bauteile 2, 12 miteinander erreicht.

Man erkennt insbesondere in der als zweites von oben dargestellten Draufsicht auf die beiden ineinander eingefügten Bauteile 12, 2, dass die Bohrung 10 nicht nur in Richtung der jeweiligen Längsrichtung L₂, L₁ als Langloch ausgebildet ist, sondern auch in einer Richtung quer dazu größer ist, als dies für ein Verbindungselement nötig wäre. Dies bedeutet, dass auch in einer Richtung senkrecht zur jeweiligen Längsrichtung L₁, L₂ eine Verschiebbarkeit möglich ist und die beiden Bauteile in jeder so eingenommenen Position aneinander befestigt werden können. Dies hat zur Folge, dass insbesondere Schwankungen in der abgetragenen Materialmenge ausgeglichen werden können.

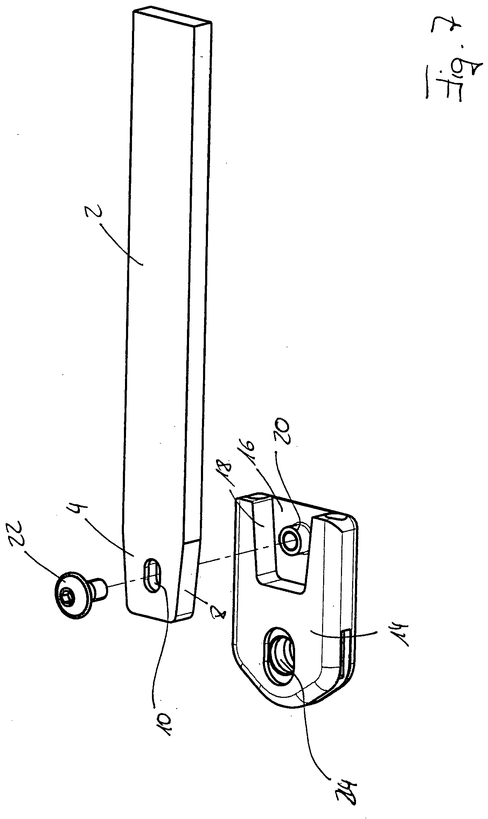

Figur 7 zeigt das zweite Bauteil 2 mit dem Verbindungsende 4 und einem daran zu befestigenden separaten Schienenkasten 14. Man erkennt ein Verbindungselement 22, das durch die Bohrung 10 des Verbindungsendes 4 geführt wird und in dem Innengewinde der Aufnahmeeinrichtung 20 eingeschraubt wird. Dadurch wird eine besonders einfache und sichere Verbindung der beiden Bauteile aneinander ermöglicht.

Der Schienenkasten 14 verfügt über eine Ausnehmung 24, durch die ein weiteres nicht gezeigtes Verbindungselement geführt werden kann, um den Schienenkasten 14 am ersten Bauelement 12 zu befestigen.

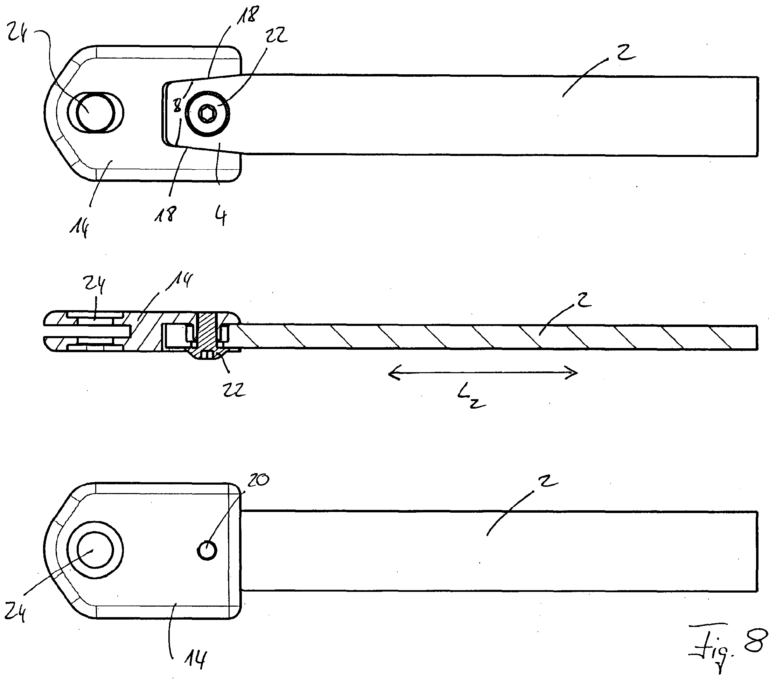

Figur 8 zeigt die in Figur 7 gezeigten Elemente im verbundenen Zustand in unterschiedlichen Ansichten. In der Mitte ist eine Schnittdarstellung entlang der Längsrichtung L₂ dargestellt. Darüber befinden sich eine Draufsicht und darunter eine Ansicht von unten. Das Verbindungselement 22 ist durch die Bohrung 10 und die Aufnahmeeinrichtung 20 hindurchgeführt und verbindet so das zweite Bauteil 2 mit dem Schienenkasten 14.

Alternativ kann durch die Ausnehmung 24 beispielsweise auch die Schwenkachse für ein Gelenk verlaufen, das in dieser besonders einfachen Weise an dem zweiten Bauteil 2 angeordnet werden kann.

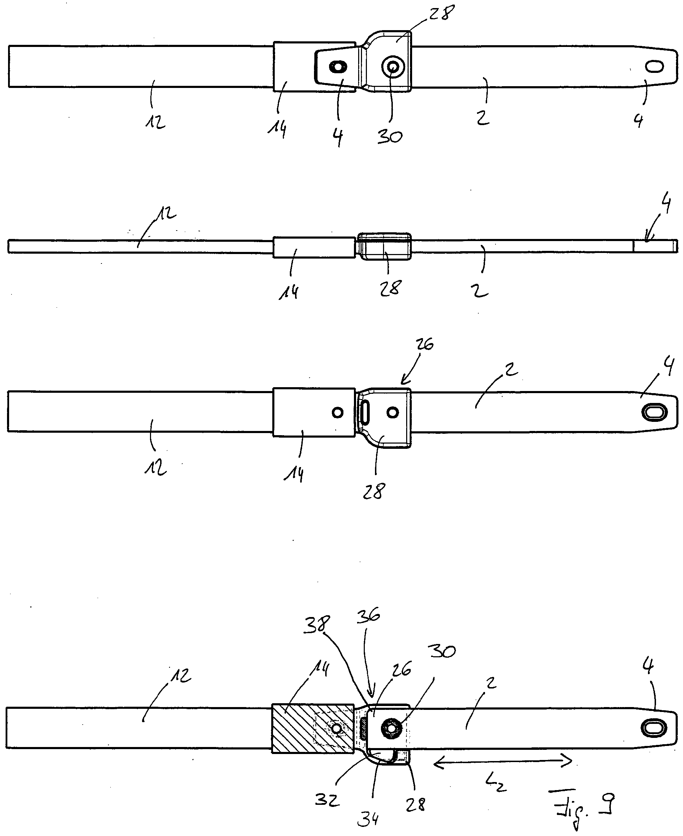

Figur 9 zeigt vier unterschiedliche Darstellungen und Ansichten des ersten Bauteils 12, an dem sich der Schienenkasten 14 befindet. Von oben nach unten sind eine Draufsicht, eine Seitenansicht, eine Ansicht von unten und eine Längsschnittdarstellung gezeigt. Im rechten Teil der jeweiligen Darstellungen befindet sich das zweite Bauteil 2 mit dem Verbindungsende 4. Im gezeigten Ausführungsbeispiel ist jedoch das zweite Bauteil 2 nicht mit dem Verbindungsende 4 sondern mit dem gegenüberliegenden Ende 26 an dem Schienenkasten 14 angeordnet. An diesem Ende ist ein separates Verbindungsendebauteil 28 angeordnet, das über ein zweites Verbindungselement 30 am Ende 26 des zweiten Bauteils 2 festgelegt ist. In der obersten Darstellung der Figur 9 ist zu erkennen, dass das Verbindungsendebauteil 28 ebenfalls ein Verbindungsende 4 aufweist, das wie das Verbindungsende 4 des zweiten Bauteils 2 ausgebildet ist. Dieses Verbindungsende 4 ist in bereits beschriebener Weise im Schienenkasten 14 des ersten Bauteils 12 angeordnet.

In der untersten Darstellung der Figur 9 ist zu erkennen, dass das Verbindungsendebauteil 28 einen Verschiebekeil 32 aufweist, der in einer dafür vorgesehenen Aufnahme in Richtung der zweiten Längsrichtung L₂ verschieblich angeordnet ist. Der Verschiebekeil 32 liegt dazu an einer Verschiebefläche 34 des Verbindungsendebauteils 28 an.

Durch ein Verschieben des Verschiebekeils 32 entlang der Verschiebefläche 34, die einen von 0° verschiedenen Winkel zur zweiten Längsrichtung L₂ aufweist, verringert sich die lichte Weite des Aufnahmeraums 36, in den das Ende 26 des zweiten Bauteils 2 aufgenommen ist. Dadurch kann das zweite Bauteil 2 innerhalb des Aufnahmeraumes 36 eingeklemmt und durch das zusätzliche zweite Verbindungselement 30 fixiert werden.

Das Ende 26 verfügt im gezeigten Ausführungsbeispiel über eine abgerundete Ecke 38. Dadurch, dass die gegenüberliegende Ecke nicht abgerundet ist und der Aufnahmeraum 36 des Verbindungsendebauteils 28 dieser Formgebung angepasst ist, kann das Ende 26 nur in einer Orientierung in den Aufnahmeraum 36 eingeführt werden. An welcher Ecke die abgerundete Ecke 38 angeordnet ist und welche tatsächliche Form diese Ecke aufweist, ist dabei für die Funktionalität unerheblich, solange durch die Formgebung eine eindeutige Unterscheidung der beiden Ecken möglich ist und der Aufnahmeraum 36 an diese Formgebung angepasst ist.

Natürlich kann statt des Verbindungsendes 4 am Verbindungsendebauteil 28 auch ein Schienenkasten 14 vorgesehen sein. Dann handelt es sich bei dem Bauteil nicht um ein Verbindungsendebauteil 28, sondern um einen separaten Schienenkasten 14, der an einem Ende eines Bauteils, das nicht als ein Verbindungsende 4 ausgebildet ist, angeordnet werden kann. Durch diese separaten Bauteile können auch herkömmliche Schienen und Bauteile nach dem Stand der Technik mit den Vorteilen der vorliegenden Erfindung ausgerüstet werden.

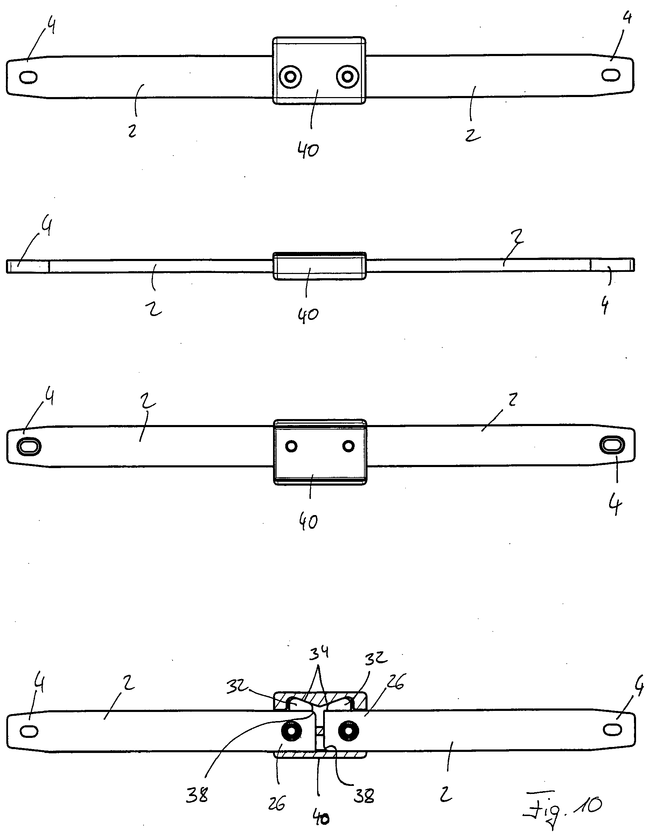

Figur 10 zeigt zwei zweite Bauteile 2 mit Verbindungsenden 4, die über ein Adapterelement 40 miteinander verbunden sind. Auch Figur 10 zeigt die bereits aus Figur 9 bekannten Ansichten. Die beiden zweiten Bauteile 2 sind über ihre Enden 26 in das Adapterelement 40 eingebracht. Dies ist besonders in der untersten Schnittdarstellung der Figur 10 deutlich zu erkennen. Das Adapterelement 40 verfügt über zwei Verschiebekeile 32 die an jeweils einer Verschiebefläche 34 verschiebbar angeordnet sind. Auf diese Weise kann das bereits aus Figur 9 bekannte Wirkprinzip erreicht werden und die Enden 26 der zweiten Bauteile 2 können in dem Adapterelement 40 eingeklemmt werden. Die beiden Enden 26 verfügen über jeweils eine abgerundete Ecke 38, die dafür sorgt, dass sie nur in einer Orientierung in das Adapterelement 40 eingebracht werden können.

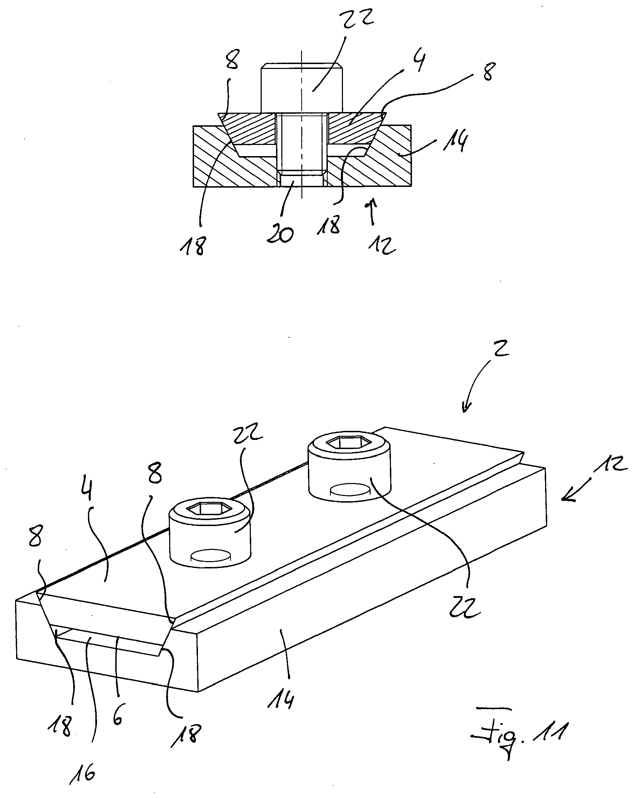

Figur 11 zeigt im unteren Bereich die schematische dreidimensionale Anordnung des ersten Bauteils 12 mit dem Schienenkasten 14 mit einem daran angeordneten zweiten Bauteil 2 mit einem Verbindungsende 4. Das Verbindungsende 4 hat zwei konisch aufeinander zulaufende Seitenwände 8, die im in Figur 11 gezeigten Zustand an den beiden Anschlagswänden 18 des Schienenkastens 14 anliegen. Man erkennt, dass im gezeigten Ausführungsbeispiel die Unterseite 6 nicht am Boden 16 anliegt. Über zwei Verbindungselemente 22, die vorliegend als Schrauben ausgebildet sind, sind die beiden Bauteile 2, 12 miteinander verbunden. Im oberen Bereich der Figur 11 ist die gleiche Anordnung in einer Schnittdarstellung gezeigt. In dieser Darstellung wird nochmals deutlich, dass die konisch aufeinander zulaufenden Seitenwände 8 an den ebenfalls konisch aufeinander zulaufenden Anschlagswänden 18 anliegen und das Verbindungselement 22 in der Aufnahmeeinrichtung 20, die im gezeigten Ausführungsbeispiel mit einem Innengewinde versehen ist, eingesetzt ist.

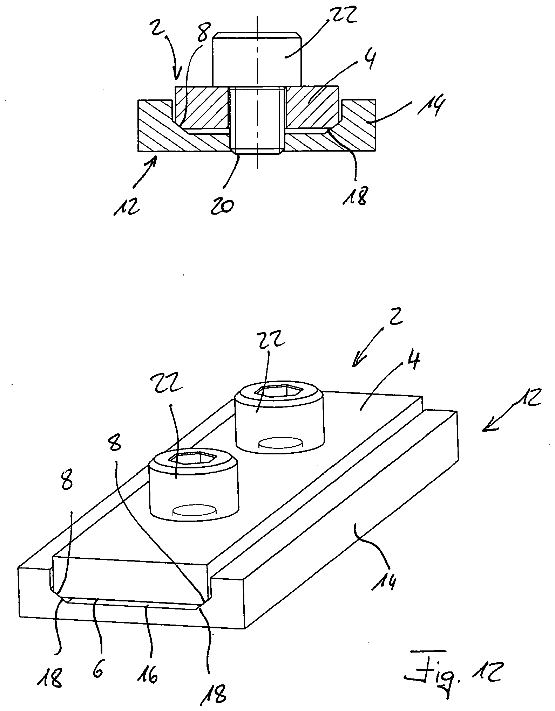

Figur 12 zeigt eine ähnliche Darstellung. Auch hier ist im unteren Teil der Figur 12 das erste Bauteil 12 mit dem Schienenkasten 14 und das zweite Bauteil 2 mit dem Verbindungsende 4 dargestellt. Im Unterschied zu der in Figur 11 gezeigten Ausführungsform nehmen die konisch aufeinander zulaufenden Seitenwände 8 und die ebenfalls konisch aufeinander zulaufenden Anschlagswände 18 nicht den gesamten Bereich der jeweiligen Flächen ihres Bauteiles 2, 12 ein. Dennoch laufen sie abschnittsweise konisch aufeinander zu und liegen im gezeigten Ausführungsbeispiel aneinander an. Auch hier wird deutlich, dass die Unterseite 6 nicht am Boden 16 anliegen muss. Im oberen Bereich der Figur 12 ist eine Schnittdarstellung durch die im unteren Teil gezeigte Verbindung gezeigt. Durch das Verbindungselement 22, das in die Aufnahmeeinrichtung 20 greift, werden die beiden Bauteile 2, 12 gegeneinander verspannt, sodass eine spielfreie und stabile Verbindung entsteht.

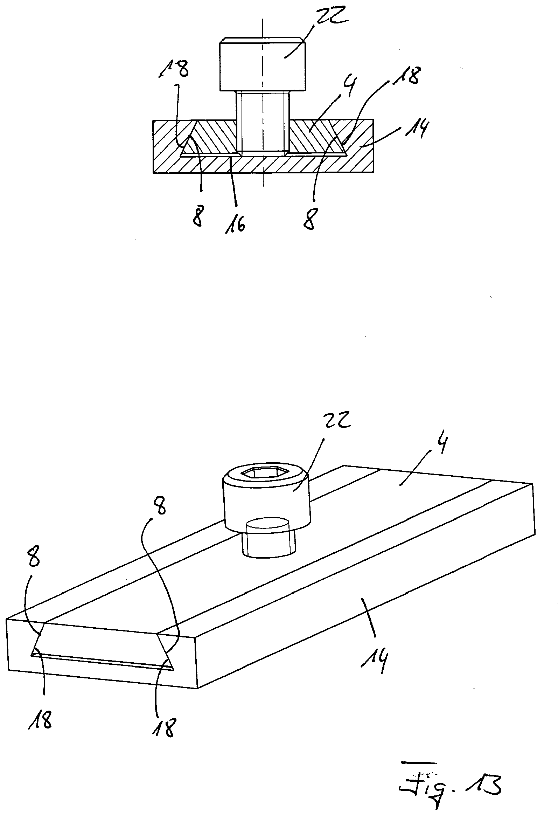

Figur 13 zeigt ein weiteres Ausführungsbeispiel des Schienenkastens 14 und des Verbindungsendes 4. Auch dieses Verbindungsende 4 verfügt über zwei konisch aufeinander zulaufende Seitenwände 8, die an ebenfalls zwei konisch aufeinander zulaufenden Anschlagswänden 18 anliegen. Allerdings ist die Richtung, in der die beiden Wände konisch aufeinander zulaufen, im Vergleich zu den in den Figuren 12 und 13 gezeigten Ausführungsbeispielen um 180° gedreht. Im in Figur 13 gezeigten Ausführungsbeispiel ist nur ein einziges Verbindungselement 22 vorgesehen, durch das die beiden Bauteile miteinander verspannt werden. Im oberen Bereich der Figur 13 ist die Situation in Form einer Schnittdarstellung gezeigt. Das Verbindungselement 22 wird in diesem Ausführungsbeispiel durch die Ausnehmung im Verbindungsende 4 des zweiten Bauteils 2 hindurchgeführt, greift jedoch nicht in eine Ausnehmung im ersten Bauteil 12 bzw. in dessen Schienenkasten 14 ein. Statt dessen liegt das Verbindungselement 22 mit dem in Figur 13 unten dargestellten Ende am Boden 16 des Schienenkastens 14 an, sodass ein weiteres Einschrauben des Verbindungselementes 22 im in Figur 13 gezeigten Ausführungsbeispiel nach unten eine Kraft zur Folge hat, die auf das zweite Bauteil 2 bzw. dessen Verbindungsende 4 wirkt und nach oben gerichtet ist. Dadurch werden die beiden Bauteile 2, 12 auch in diesem Beispiel gegeneinander verspannt.

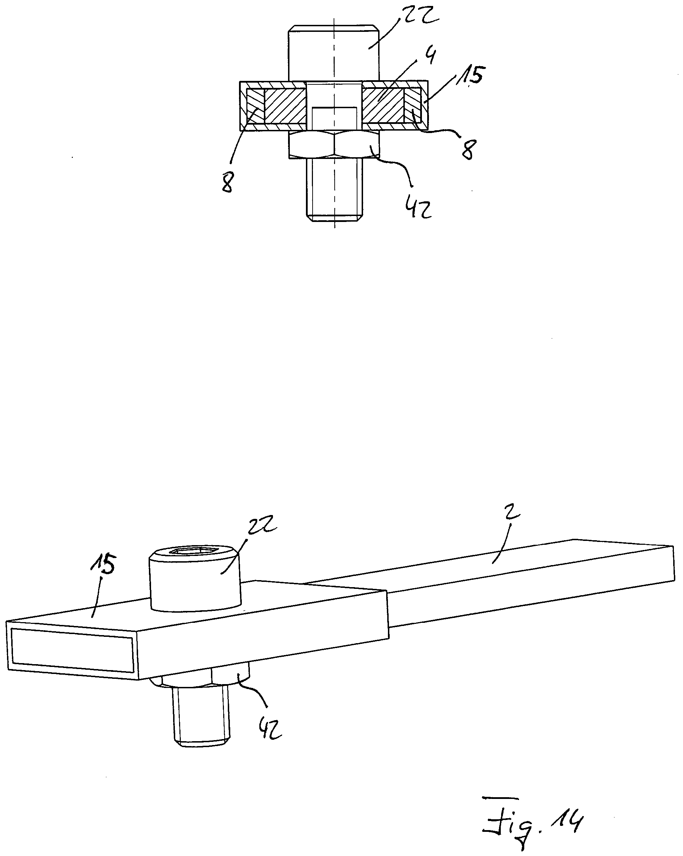

Figur 14 zeigt im unteren Abschnitt ein zusätzliches Element 15, das als Schloss bezeichnet wird und in das ein Verbindungsende 4 des zweiten Bauteiles 2 eingefügt ist. Das zusätzliche Element 15 kann fest mit dem zweiten Bauteil 2 verbunden sein oder als separates Bauteil ausgebildet sein. Statt der in Figur 14 gezeigten umlaufend geschlossenen Ausführung kann das zusätzliche Element 15 auch beispielsweise aus zwei oben und unten anzuordnenden Plattenelementen bestehen. Das zusätzliche Element 15 ist umlaufend geschlossen ausgebildet, sodass es das nicht dargestellte Verbindungsende 4 des zweiten Bauteils 2 vollständig umgibt. Auch hier wird über ein Verbindungselement 22, das wieder als Schraube ausgebildet ist, die Verbindung zwischen den beiden Bauteilen 2, 12 arretiert. Im in Figur 14 gezeigten Ausführungsbeispiel ist auf dem Verbindungselement 22 eine Mutter 42 angeordnet, durch die die benötigte Kraft aufgebracht wird.

Im oberen Bereich der Figur 14 ist wieder eine Schnittdarstellung gezeigt, bei der deutlich erkennbar ist, dass das zusätzliche Element 15 umlaufend geschlossen ausgebildet ist. Das Verbindungselement 22 mit der daran angeordneten Mutter 42 verläuft sowohl durch das zusätzliche Element 15 als auch durch das Verbindungsende 4 des zweiten Bauteils 2. Man erkennt durch eine andere Schraffur angedeutet die beiden aufeinander konisch zulaufenden Seitenwände 8, die analog zu dem beispielsweise in Figur 1 dargestellten Bauteil 2 verlaufen.

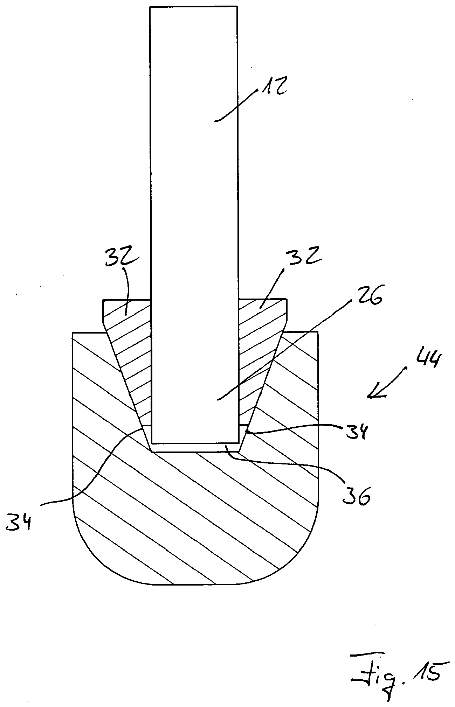

Figur 15 zeigt ein Funktionsbauteil 44, das auf das Ende 26 eines Bauteiles 12 aufgesetzt ist. Dazu verfügt das Funktionsbauteil 44 über den Aufnahmeraum 36, in dem vorliegend zwei Verschiebekeile 32 angeordnet sind. Diese können im in Figur 15 gezeigten Ausführungsbeispiel nach oben und unten verschoben werden und sorgen so dafür, dass das Bauteil 12 im Aufnahmeraum 36 festgeklemmt wird, sodass es durch ein in Figur 15 nicht gezeigtes Verbindungselement befestigt werden kann. Das Funktionsbauteil 44 verfügt über zwei Verschiebeflächen 34, entlang derer die beiden Verschiebekeile 32 verschoben werden können. Insbesondere für den Fall, dass nur ein Verschiebekeil 32 vorhanden ist, kann dieser mit dem zusätzlichen Element 15, wie es in Figur 14 dargestellt ist, verbunden sein. Dann wird mit dem Aufschieben des Elementes 15 die Klemmung erreicht.

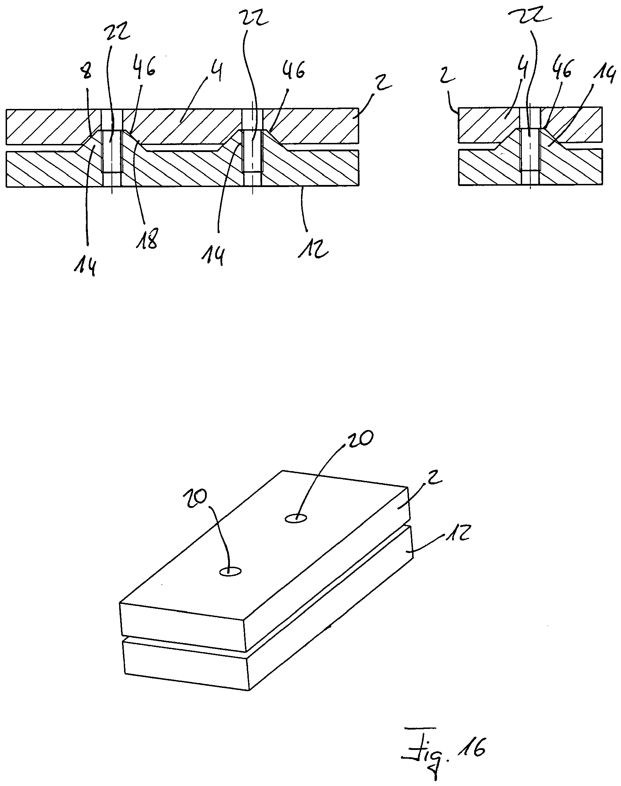

Figur 16 zeigt im unteren Bereich ein weiteres Ausführungsbeispiel zweier Bauteile, die erfindungsgemäß miteinander verbunden sind. Im unteren Bereich der Figur 16 ist eine 3D-Ansicht dargestellt, in der das erste Bauteil 12 und das zweite Bauteil 2 dargestellt ist. Im zweiten Bauteil 2 sind zwei Aufnahmeeinrichtungen 20 dargestellt, durch die Verbindungselemente 22 geführt werden können. Erst in den im oberen Bereich von Figur 16 dargestellten Schnittdarstellungen ist zu erkennen, wie die beiden Bauteile spielfrei aneinander gelagert werden können. Dazu verfügt das erste Bauteil 12 über Aufnahmeelemente 14, die jedoch in diesem Ausführungsbeispiel nicht als Schienenkasten, sondern als kegelstumpfförmige Erhebungen ausgebildet sind. Dabei ist es denkbar, nur eine einzige dieser Aufnahmeeinrichtungen 14 vorzusehen, wie dies im rechten oberen Teil der Figur 16 dargestellt ist, oder mehr als eine, beispielsweise zwei kegelförmige Erhebungen an dem ersten Bauteil 12 anzuordnen, wie dies im linken oberen Bereich der Figur 16 dargestellt ist. Das zweite Bauteil 2 verfügt am Verbindungsende 4 über zu den Kegelstümpfen korrespondierend ausgebildete Vertiefungen 46, in die die kegelstumpfförmigen Erhebungen eingreifen können. Die Seitenwände dieser Vertiefungen 46 bilden dabei die Seitenwände 8, die wie in den Schnittdarstellungen in Figur 16 gut zu erkennen ist, konisch aufeinander zulaufen. Die Seitenwände der kegelstumpfförmigen Erhebungen 14 am ersten Bauteil 12 bilden die konisch aufeinander zulaufenden Anschlagswände 18. In die durch beide Bauteile 2, 12 durchgehende Bohrung sind Verbindungselemente 22 eingesetzt, die beispielsweise als Schraube ausgebildet sind.

Ein Orthopädietechniker, der zwei Bauteile 2, 12 mit der in Figur 16 gezeigten Ausführungsform miteinander verbinden soll, kann dies auf besonders einfache Weise tun. So kann beispielsweise herstellerseitig lediglich das erste Bauteil 12 mit dem kegelstumpfförmigen Erhebungen 14 versehen sein, während das erste Bauteil 2 ohne Vertiefungen oder Bohrungen geliefert wird. Die beiden Bauteile 2, 12 können besonders einfach insbesondere nach einem Ablängen des zweiten Bauteils 12 aneinander angelegt werden, sodass nun durch die Bohrungen, die im ersten Bauteil 12 enthalten sind, an exakt den richtigen Positionen auch im zweiten Bauteil 2 entsprechende Bohrungen hergestellt werden können. An den so definierten Stellen können auch die kegelstumpfförmigen Vertiefungen 46 in das zweite Bauteil 2 eingebracht werden, sodass eine exakte Positionierung der Vertiefungen 46 relativ zu den Positionen der kegelstumpfförmigen Erhebung möglich ist.

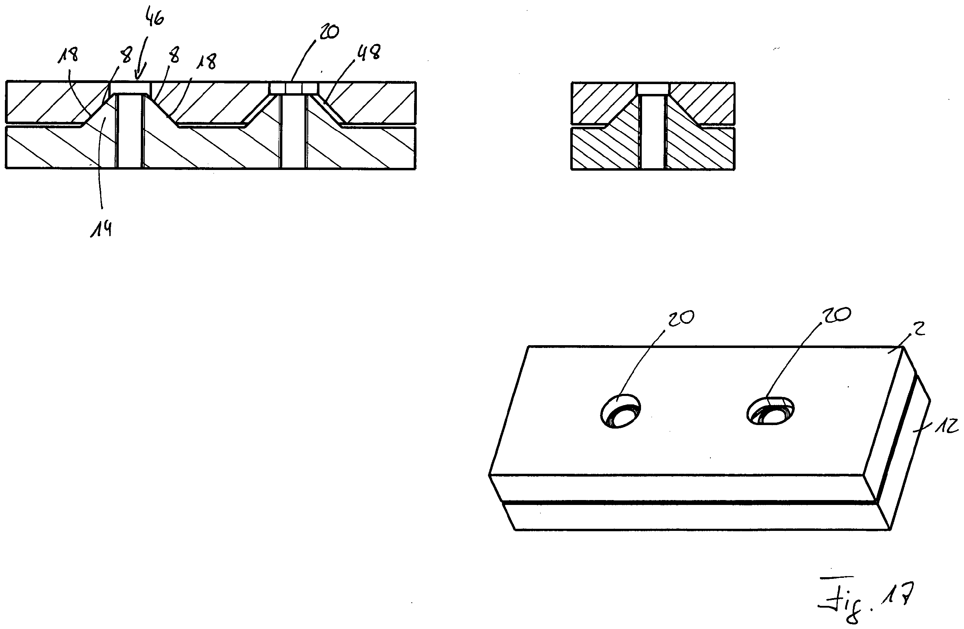

Figur 17 zeigt eine ähnliche Ausführungsform, bei der man insbesondere in der unten dargestellten 3D-Ansicht erkennt, dass eine der Aufnahmeeinrichtungen 20 als Langloch ausgebildet ist. In der im oberen linken Bereich der Figur 17 dargestellten Schnittdarstellung wird deutlich, was damit erreicht wird. Während die linke Erhebung 14 mit ihren Anschlagswänden 18 spielfrei an den konisch aufeinander zulaufenden Seitenwänden 8 der Vertiefung 46 anliegt, ist dies bei der rechten Erhebung nicht der Fall. Hier bildet sich ein Spalt 48, durch den Fertigungstoleranzen ausgeglichen werden können. Die rechte Erhebung dient nur als Verdrehsicherung, durch die eine Rotation um die Längsachse der linken Erhebung 14 verhindert werden soll. Daher ist die dort gezeigte Aufnahmeeinrichtung 20 als Langloch ausgebildet, um hier Fertigungstoleranzen ausgleichen und dennoch ein Verbindungselement 22 einführen zu können. Im rechten oberen Abschnitt der Figur 17 ist eine Schnittdarstellung gezeigt, die im Wesentlichen der in Figur 16 oben rechts gezeigten Darstellung entspricht.

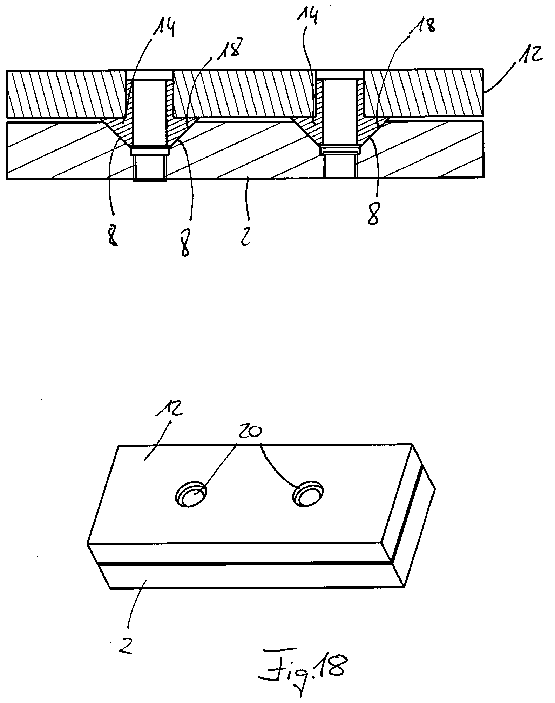

Figur 18 zeigt eine weitere Ausführungsform, die im Wesentlichen der in Figur 16 gezeigten Ausführungsform entspricht. Im unteren Bereich ist wieder eine 3D-Ansicht mit dem ersten Bauteil 12, dem zweiten Bauteil 2 sowie zwei Aufnahmeeinrichtungen 20 dargestellt. Anders als bei den bisher gezeigten Darstellungen befindet sich nun jedoch das erste Bauteil 12 oben. In der im oberen Teil von Figur 18 dargestellten Schnittdarstellung erkennt man, dass das erste Bauteil 12 und die beiden dargestellten kegelstumpfförmigen Erhebungen, die die Aufnahmeelemente 14 bilden, nicht einstückig ausgebildet sind, sondern dass die Aufnahmeelemente 14 beispielsweise über Schraubverbindungen in das erste Bauteil 12 eingelassen sind. Das zweite

Bauteil 2 verfügt wieder über entsprechende Vertiefungen 46, die mit ihren Seitenwänden 8 spielfrei an den konisch zulaufenden Anschlagswänden 18 der Aufnahmeelemente 14 anliegen.

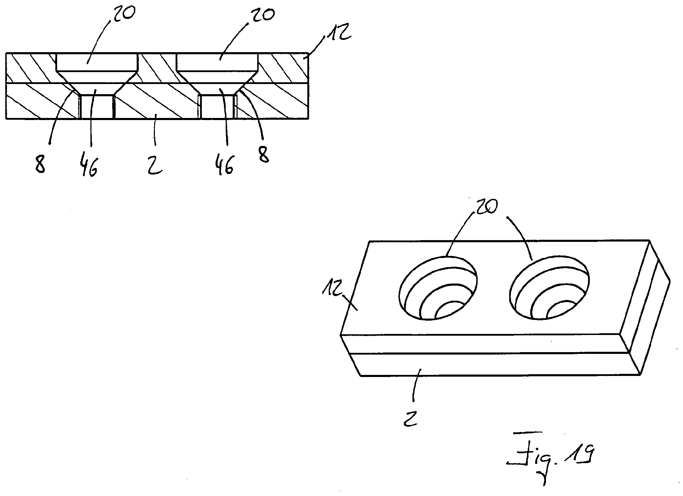

Figur 19 zeigt eine weitere Ausführungsform, bei der sich die 3D-Ansicht im unteren Bereich von Figur 19 von der in Figur 18 gezeigten 3D-Ansicht im Wesentlichen durch die Größe der Aufnahmeeinrichtungen 20 unterscheidet. In der Schnittdarstellung im oberen Bereich von Figur 19 ist zu erkennen, dass das zweite Bauteil 2, wie in den Figuren 16 bis 18 bereits gezeigt, über Vertiefungen 46 verfügt. Diese Vertiefungen 46 verfügen wieder über Seitenwände 8, die konisch aufeinander zulaufen. Das erste Bauteil 12 ist in Figur 19 in der Schnittdarstellung nur mit den Aufnahmeeinrichtungen 20 dargestellt, deren Seitenwände die Seitenwände 8 der Vertiefungen 46 nahtlos fortsetzen. In diese Aufnahmeeinrichtungen 20 können nun Kegelschrauben eingesetzt werden, die beispielsweise einen kegelstumpfförmigen Kopf aufweisen, dessen Mantelfläche die Anschlagswände 18 bildet. Auch diese verlaufen konisch aufeinander zu, sodass auch diese Ausführungsform erfindungsgemäß ist.

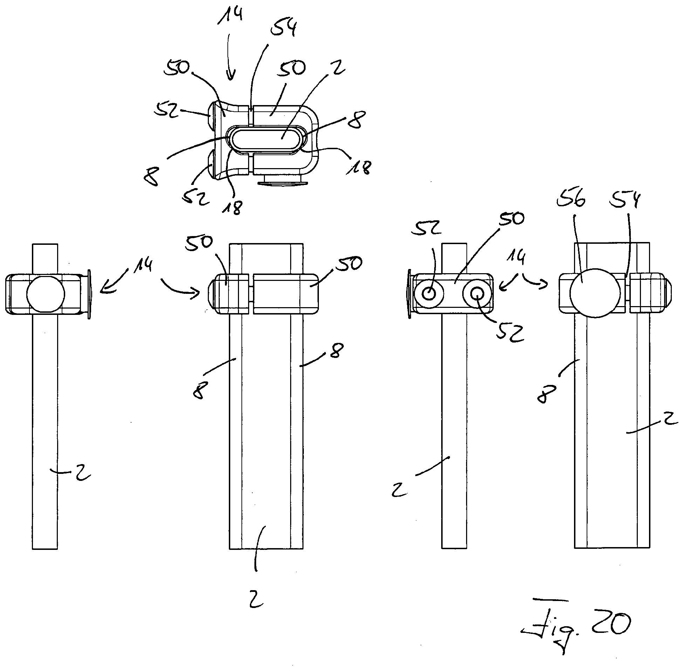

Figur 20 zeigt im unteren Bereich das zweite Bauteil 2 in vier unterschiedlichen Seitenansichten. Darüber ist eine Schnittdarstellung gezeigt, die eine Blickrichtung entlang der Längsrichtung des zweiten Bauteils 2 zeigt. Insbesondere in dieser Schnittdarstellung ist zu erkennen, dass das zweite Bauteil 2 am Verbindungsende 4 über einen ovalen Querschnitt verfügt. Die Seitenwände 8 verlaufen folglich gekrümmt und laufen daher von der Mitte, also der breitesten Stelle des zweiten Bauteils 2 ausgehend, in Figur 20 im oberen Abschnitt nach oben und unten gesehen aufeinander zu. Die Breite des zweiten Bauteils 2 nimmt in dieser Richtung von der Mitte aus gesehen kontinuierlich ab, sodass die Seitenwände 8 aufeinander zulaufen.

Auf das zweite Bauteil 2 ist das Aufnahmeelement 14 aufgesteckt, das im in Figur 20 gezeigten Ausführungsbeispiel aus zwei Klemmelementen 50 besteht. Diese sind durch zwei Schrauben 52 miteinander verbunden, wobei zwischen den beiden Klemmelementen 50 ein Zwischenraum 54 liegt, dessen Breite durch Ein- bzw. Ausschrauben der Schrauben 52 verkleinert bzw. vergrößert werden kann. Die beiden Klemmelemente 50 bilden eine Ausnehmung, die zumindest auf einer Seite schräg aufeinander zulaufende Anschlagswände 18 aufweist, wie dies beispielsweise im oberen Bereich der Figur 20 zu erkennen ist. Werden die Schrauben 52 betätigt, werden die beiden Klemmelemente 50 aufeinander zu bewegt und klemmen so das zweite Bauteil 2 spielfrei ein.

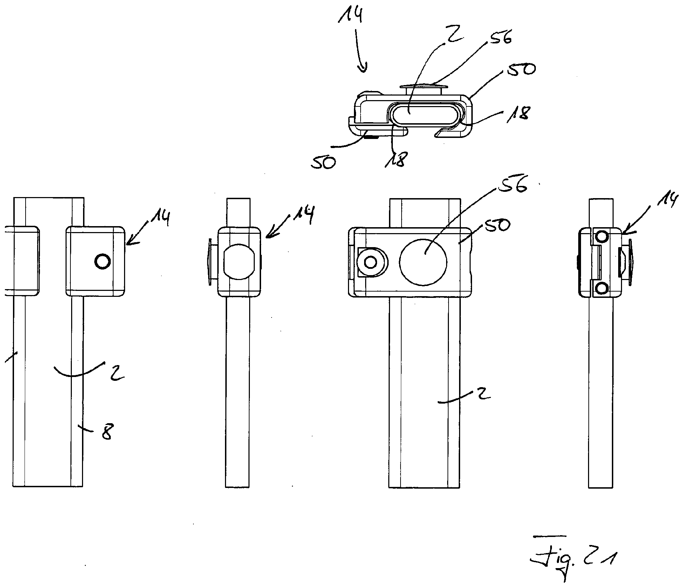

Figur 21 zeigt eine ähnliche Darstellung, wobei auch hier auf das zweite Bauteil 2 ein aus zwei Klemmelementen 50 bestehendes Aufnahmeelement 14 aufgesteckt ist. Das im oberen Bereich von Figur 21 in der Schnittdarstellung unten links dargestellte Klemmelement 50 ist im in Figur 21 gezeigten Ausführungsbeispiel nach links und rechts verschieblich. Es verfügt genauso wie das zweite Klemmelement 50 über jeweils eine schräg verlaufende Anschlagswand 18, die konisch aufeinander zulaufen. Durch Verschieben des Klemmelementes 50 relativ zum zweiten Klemmelement 50 werden die beiden Anschlagswände 18 aufeinander zu bzw. voneinander weg bewegt, sodass es zum Einklemmen des zweiten Bauteils 2 und damit zu einer spielfreien Verbindung kommt. Sowohl in Figur 20 als auch in Figur 21 verfügt das Aufnahmeelement 14 über ein kreisförmig dargestelltes Befestigungselement 56, an dem beispielsweise Gurte befestigt werden können, die um den Körper eines Trägers eines derartigen Schienensystems herumgelegt werden können.

### Bezugszeichenliste

- L₁: erste Längsrichtung
- L₂: zweite Längsrichtung
- 2: zweites Bauteil
- 4: Verbindungsende
- 6: Unterseite
- 8: Seitenwand
- 10: Bohrung
- 12: erstes Bauteil
- 14: Schienenkasten
- 16: Boden
- 18: Anschlagswand
- 20: Aufnahmeeinrichtung
- 22: Verbindungselement
- 24: Ausnehmung
- 26: Ende
- 28: Verbindungsendebauteil
- 30: zweites Verbindungselement
- 32: Verschiebekeil
- 34: Verschiebefläche
- 36: Aufnahmeraum
- 38: abgerundete Ecke
- 40: Adapterelement
- 42: Mutter
- 44: Funktionsbauteil
- 46: Vertiefung
- 48: Spalt
- 50: Klemmelement
- 52: Schrauben
- 54: Zwischenraum
- 56: Befestigungselement
Fr/bro/ne-rog

## Patentansprüche

1. Orthopädietechnisches Schienensystem, das
- ein erstes Bauteil (12) mit einem Aufnahmeelement (14) und
- ein zweites Bauteil (2) mit einem Verbindungsende (4) zum Verbinden mit dem Aufnahmeelement (14) aufweist,
wobei
- das Aufnahmeelement (14) zwei einander gegenüberliegende Anschlagswände (18) aufweist,
- das Verbindungsende (4) zwei einander gegenüberliegende Seitenwände (8) aufweist und
- das Aufnahmeelement (14) und das Verbindungsende (4) durch wenigstens ein Verbindungselement (22) miteinander verbindbar sind, sodass im verbundenen Zustand die Seitenwände (8) an den Anschlagswänden (18) anliegen,
wobei die Anschlagswände (18) und die Seitenwände (8) jeweils konisch aufeinander zulaufen und das Aufnahmeelement (14) und das Verbindungsende (4) stufenlos zueinander positionierbar sind, **dadurch gekennzeichnet, dass** das Aufnahmeelement (14) und das Verbindungsende (4) stufenlos so miteinander verbindbar sind, dass sie auch in der Position miteinander verbindbar sind, in der sie spielfrei aneinander anliegen, wobei das Schienensystem zum Verbinden des Aufnahmeelementes (14) mit dem Verbindungsende (4) wenigstens ein Verbindungselement (22) aufweist, das durch ein Langloch in dem ersten Bauteil (12) oder dem zweiten Bauteil (2) geführt ist.

2. Schienensystem nach Anspruch 1, **dadurch gekennzeichnet, dass** das Aufnahmeelement (14) einen Boden (16) und das Verbindungsende (4) eine Unterseite (6) aufweist, wobei die Unterseite (6) im verbundenen Zustand an dem Boden (16) anliegt.

3. Schienensystem nach einem der vorstehenden Ansprüche, **dadurch gekennzeichnet, dass** das erste Bauteil (12) eine erste Längsrichtung (L₁) aufweist und wenigstens eine Anschlagswand (18) mit der ersten Längsrichtung (L₁) einen von 0° verschiedenen Winkel einschließt.

4. Schienensystem nach einem der vorstehenden Ansprüche, **dadurch gekennzeichnet, dass** das zweite Bauteil (2) eine zweite Längsrichtung (L₂) aufweist und wenigstens eine Seitenwand (8) mit der zweiten Längsrichtung (L₂) einen von 0° verschiedenen Winkel einschließt.

5. Schienensystem nach einem der Ansprüche 3 oder 4, **dadurch gekennzeichnet, dass** beide Anschlagswände (18) oder beide Seitenwände (8) oder sowohl beide Anschlagswände (18) als auch beide Seitenwände (8) mit der jeweiligen ersten Längsrichtung (L₁) bzw. der zweiten Längsrichtung (L₂) einen von 0° verschiedenen Winkel einschließen.

6. Schienensystem nach Anspruch 5, **dadurch gekennzeichnet, dass** die Winkel zwischen den Anschlagswänden (18) und der ersten Längsrichtung (L₁) sowie zwischen den Seitenwänden (8) und der zweiten Längsrichtung (L₂) identisch sind.

7. Schienensystem nach einem der vorstehenden Ansprüche, **dadurch gekennzeichnet, dass** der Schienenkasten (14) und das Verbindungsende (4) in zwei senkrecht aufeinander stehenden Richtungen stufenlos zueinander positionierbar und miteinander verbindbar sind.

8. Schienensystem nach einem der vorstehenden Ansprüche, **dadurch gekennzeichnet, dass** das Aufnahmeelement (14) lösbar mit dem ersten Bauteil (2) verbunden ist.

## Claims

1. An orthopedic rail system which has
- a first component (12) with a receiving element (14) and
- a second component (2) with a connection end (4) for connecting to the receiving element (14),
wherein
- the receiving element (14) has two stop walls (18) lying opposite each other,
- the connection end (4) has two lateral walls (8) lying opposite each other, and
- the receiving element (14) and the connection end (4) are connectable to each other by at least one connection element (22), such that the lateral walls (8) bear on the stop walls (18) in the connected state,
wherein the stop walls (18) and the lateral walls (8), respectively, taper conically toward each other and the receiving element (14) and the connection end (4) are able to be positioned steplessly relative to each other, **characterized in that** the receiving element (14) and the connection end (4) are able to be positioned steplessly relative to each other in such a way, that they are connectable to each other particularly in the position in which they bear on each other in a manner free of play, wherein the rail system has at least one connection element (22) for connecting the receiving element (14) to the connection end (4), which is guided through an oblong hole in the first component (12) or the second component (2).

2. The rail system as claimed in claim 1, **characterized in that** the receiving element (14) has a base (16) and the connection end (4) has an underside (6), wherein the underside (6) bears on the base (16) in the connected state.

3. The rail system as claimed in one of the preceding claims, **characterized in that** the first component (12) has a first longitudinal direction (L₁), and at least one stop wall (18) encloses, with the first longitudinal direction (L₁), an angle different than 0°.

4. The rail system as claimed in one of the preceding claims, **characterized in that** the second component (2) has a second longitudinal direction (L₂), and at least one lateral wall (8) encloses, with the second longitudinal direction (L₂), an angle different than 0°.

5. The rail system as claimed in either of claims 3 and 4, **characterized in that** both stop walls (18) or both lateral walls (8) or both stop walls (18) and also both lateral walls (8) enclose, with the respective first longitudinal direction (L₁) or second longitudinal direction (L₂), an angle different than 0°.

6. The rail system as claimed in claim 5, **characterized in that** the angles between the stop walls (18) and the first longitudinal direction (L₁) and between the lateral walls (8) and the second longitudinal direction (L₂) are identical.

7. The rail system as claimed in one of the preceding claims, **characterized in that** the rail box (14) and the connection end (4) are able to be positioned steplessly relative to each other in two mutually perpendicular directions and are connectable to each other.

8. The rail system as claimed in one of the preceding claims, **characterized in that** the receiving element (14) is connected releasably to the first component (2).

## Revendications

1. Système d'attelle orthopédique comportant
- un premier composant (12) pourvu d'un élément de réception (14), et
- un second composant (2) présentant une extrémité de liaison (4) pour la liaison avec l'élément de réception (14),
dans lequel
- l'élément de réception (14) comprend deux parois de butée (18) opposées l'une à l'autre,
- l'extrémité de liaison (4) comprend deux parois latérales (8) opposées l'une à l'autre, et
- l'élément de réception (14) et l'extrémité de liaison (4) sont susceptibles d'être reliés l'un à l'autre par au moins un élément de liaison (22), de sorte que dans l'état relié, les parois latérales (8) prennent appui contre les parois de butée (18),
les parois de butée (18) de même que les parois latérales (8) convergeant coniquement l'une vers l'autre, et l'élément de réception (14) et l'extrémité de liaison (4) sont susceptibles d'être positionnés en continu l'un par rapport à l'autre,
**caractérisé en ce que**
l'élément de réception (14) et l'extrémité de liaison (4) sont susceptibles d'être reliés en continu l'un à l'autre de manière à pouvoir être reliés l'un à l'autre même dans la position dans laquelle ils s'appuient sans jeu l'un contre l'autre, et
pour relier l'élément de réception (14) à l'extrémité de liaison (4), le système d'attelle comprend au moins un élément de liaison (22) qui est mené à travers un trou oblong dans le premier composant (12) ou dans le second composant (2).

2. Système d'attelle selon la revendication 1,
**caractérisé en ce que**
l'élément de réception (14) présente un fond (16) et l'extrémité de liaison (4) présente une face inférieure (6), la face inférieure (6) prenant appui contre le fond (16) dans l'état relié.

3. Système d'attelle selon l'une des revendications précédentes,
**caractérisé en ce que**
le premier composant (12) présente une première direction longitudinale (L₁), et au moins une paroi de butée (18) définit un angle différent de 0° avec la première direction longitudinale (L₁).

4. Système d'attelle selon l'une des revendications précédentes,
**caractérisé en ce que**
le second composant (2) présente une seconde direction longitudinale (L₂), et au moins une paroi latérale (8) définit un angle différent de 0° avec la seconde direction longitudinale (L₂).

5. Système d'attelle selon l'une des revendications 3 ou 4,
**caractérisé en ce que**
les deux parois de butée (18) ou les deux parois latérales (8) ou encore aussi bien les deux parois de butée (18) que les deux parois latérales (8) définissent un angle différent de 0° respectivement avec la première direction longitudinale (L₁) et avec la seconde direction longitudinale (L₂).

6. Système d'attelle selon la revendication 5,
**caractérisé en ce que**
les angles entre les parois de butée (18) et la première direction longitudinale (L₁) et entre les parois latérales (8) et la seconde direction longitudinale (L₂) sont identiques.

7. Système d'attelle selon l'une des revendications précédentes,
**caractérisé en ce que**
le caisson d'attelle (14) et l'extrémité de liaison (4) sont susceptibles d'être positionnés en continu l'un par rapport à l'autre et d'être reliés l'un à l'autre dans deux directions perpendiculaires l'une à l'autre.

8. Système d'attelle selon l'une des revendications précédentes,
**caractérisé en ce que**
l'élément de réception (14) est relié de façon détachable au premier composant (2).
